# EUROPEAN PATENT SPECIFICATION

(11) **EP 4 504 143 B1**
(45) Date of publication and mention of the grant of the patent: **15.07.2026**
(21) Application number: 23715088.3
(22) Date of filing: 24.03.2023
(51) Int. Cl.: A61K 8/73, A61Q 11/00, A61K 8/34, A61K 8/46, A61K 8/86, A61K 8/33

(54) **A NON AQUEOUS ORAL CARE COMPOSITION BASED ON CARRAGEENAN, POLYOL AND A POLYETHYLENE GLYCOL AND/OR POLYPROPYLENE GLYCOL**
NICHTWÄSSRIGE MUNDPFLEGEZUSAMMENSETZUNG AUF BASIS VON CARRAGEEN, POLYOL UND EINEM POLYETHYLENGLYKOL UND/ODER POLYPROPYLENGLYKOL
COMPOSITION NON AQUEUSE D'HYGIÈNE BUCCALE À BASE DE CARRAGHÉNANE, DE POLYOL ET DE POLYÉTHYLÈNE GLYCOL ET/OU DE POLYPROPYLÈNE GLYCOL.

(30) Priority: 01.04.2022 EP 22166278
(43) Date of publication of application: 12.02.2025
(73) Proprietor: Unilever IP Holdings B.V., 6708 WH Wageningen (NL); Unilever Global IP Limited, Wirral, Merseyside CH62 4ZD (GB)
(72) Inventor: BOTHA, Johannes Andries, 6708 WH Wageningen (NL); LITTLEWOOD, David Thomas, 6708 WH Wageningen (NL); RILEY, Robert George, 6708 WH Wageningen (NL)
(74) Representative: Unilever Patent Group
(86) International application number: PCT/EP2023/057709
(87) International publication number: WO 2023/186746

(56) References cited:
- CN-A- 104 027 260
- US-A- 4 585 648
- US-A1- 2003 124 067
- US-A1- 2012 020 898
- US-A1- 2012 064 017
- US-A1- 2012 315 228

## Description

### Field of the Invention

The present invention is concerned with non-aqueous oral care compositions.

### Background of the Invention

Oral care compositions such as dentifrices typically contain dentally acceptable abrasive, humectant, water, and water-soluble polymer which serves as a thickener and binder for the ingredients. A variety of other ingredients such as flavours, sweeteners, preservatives and fluoride are also utilized at low levels.

However, there are many materials which are physically or chemically incompatible with the aqueous environments found in typical dentifrice formulations.

Non-aqueous formulations have been suggested as a way of improving the stability of these materials. For example, WO96/03108 describes a non-aqueous dentifrice composition comprising a carboxyvinyl polymer, a humectant, a polyethylene glycol and a dentally acceptable abrasive.

EP 2585031 discloses a non-aqueous oral care composition with a liquid continuous phase comprising a thickening agent, a humectant, and one or more liquid polyethylene glycols having a melting point below 25°C, in which the liquid continuous phase is structured with crystals of one or more solid polyethylene glycols having a melting point of 25°C or above.

US2012/020898 and US2003/124067 disclose compositions comprising carrageenan. US2012/315228 discloses compositions based on more than 50 wt % glycerin, SLS as sole anionic surfactant and mixtures of a calcium silicate and a phosphate source. Those compositions neither comprise carrageenan nor comprise PEG/PPG.

However there remains the need for non-aqueous compositions with a good viscosity profile and flow characteristics as well as excellent storage characteristics.

It is thus an object of the present invention to provide for an oral care composition with good paste shape and storage stability.

### Description of the Invention

A first aspect of the invention relates to a non-aqueous oral care composition comprising a non-aqueous oral care composition comprising:
i) greater than 50 wt% of an organic polyol having 3 or more hydroxyl groups in the molecule;
ii) a carrageenan;
iii) a polyethylene glycol and/or polypropylene glycol having a melting point below 40°C;
iv) a mixture of a calcium silicate and a phosphate source which, when delivered to the teeth results in the *in situ* generation of hydroxyapatite on teeth.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

Any ingredients mentioned in this application that are natural or naturally derived have been sourced from Europe.

The composition of the invention is non-aqueous. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" means that water is present in an amount no greater than 5%, more preferably no greater than 3% , most preferably no greater than 1 % by weight based on the total weight of the composition.

Compositions according to the invention comprise carrageenan preferably at weight ratios of 1:2 to 2:1. iota to kappa carrageenan. The carrageenan present in the composition of the invention consists rom 33wt% to 66wt% of the total level of iota carrageenan and 33 wt% to 66 wt% of kappa carrageenan.

The total level of carrageenan is from 0.05 to 1 wt% of the total composition, more preferably 0.08 to 0.5 wt% most preferably 0.05 to 0.25 wt%.

The composition of the invention comprise polyethylene glycol or polypropylene glycol having a melting point below 40°C, more preferably below 25°C. In a preferred embodiment of the invention the melting point is greater than -20°C and ranges from -15 to 22°C, more preferably from -5 to 8°C, most preferably from 4 to 8°C.

Preferred liquid polyethylene glycols have an average value of n in the general formula H(OCH₂CH₂)ₙ OH (as described above) ranging from about 4 to 12, more preferably about 6 to 8, most preferably about 8. The average molecular weight suitably ranges from about 190 to 630, more preferably from about 285 to 420, most preferably from about 380 to 420 g/mol. Suitable commercially available materials include for example PEG 400 (ex BDH Chemicals). Mixtures of any of the above described materials may also be used.

The amount of polyethylene glycol of polyethylene glycol and/or polypropylene glycol iii (as defined above) in compositions of the invention suitably ranges from 0.1 to 10 wt% of the total composition, more preferably 0.5 to 7wt% most preferably 1 to 5 wt% on the total weight of the composition.

The preferred ratio of polyethylene glycol and/or polypropylene glycol iii) to carrageenan is from 1: 200 to 1:4, preferably from 1:1 to 1:40.

Compositions of the invention comprise organic polyols having 3 or more hydroxyl groups in the molecule (hereinafter termed "organic polyols"). Examples of such materials include glycerol, sorbitol, xylitol, mannitol, lactitol, maltitol, erythritol, and hydrogenated partially hydrolyzed polysaccharides. The most preferred organic polyol is glycerol. Mixtures of any of the above described materials may also be used.

The level of organic polyol will depend on the particular type chosen, but generally ranges from about 20 to 90% by weight based on the total weight of the composition, the amount of organic polyol suitably ranges from 35 to 85%, more preferably from 65 to 80% by total weight organic polyol based on the total weight of the composition. Ideally the composition of the invention is glycerol-based (i.e glycerol makes up a higher portion of the composition's weight than any other compound) and contains from 55 to 80% by weight glycerol based on the total weight of the composition.

The composition of the invention is used to preferably used to clean the surfaces of the oral cavity and is known as an oral care composition.

Accordingly, preferred product forms for compositions of the invention are those which are suitable for brushing and/or rinsing the surfaces of the oral cavity.

The composition of the invention is most preferably in the form of a toothpaste. Such a composition is not intentionally swallowed for purposes of systemic administration of therapeutic agents, but is applied to the oral cavity, used to treat the oral cavity and then expectorated. Typically such a composition is used in conjunction with a cleaning implement such as a toothbrush, usually by applying it to the bristles of the toothbrush and then brushing the accessible surfaces of the oral cavity.

Preferably the dentifrice/toothpaste is in the form of an extrudable semi-solid such as a cream, paste or gel (or mixture thereof).

A composition according to the invention (such as a dentifrice/toothpaste) will generally contain further ingredients to enhance performance and/or consumer acceptability, in addition to the ingredients specified above.

Compositions according to the invention, particularly toothpastes, preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials.

Preferably the composition, particularly a toothpaste, comprises a silica based abrasive. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 m²/g and an oil absorption of about 70 - 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Evonik, Sorbosil AC 77 ex PQ Corporation (having an R.I. of approximately 1.440). The amount of these silicas in the composition generally ranges from 5-60% by weight, usually 5-20% by weight.

The composition, particularly if a toothpaste preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Compositions according to the invention preferably comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

If the composition is a toothpaste it may be a dual phase paste, with the whitening pigments present in one phase.

Compositions according to the invention may comprise water-soluble or sparingly water-soluble sources of metal salts Preferred are zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; als preferred are stannous ions such as stannous fluoride and stannous chloride.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof;

Compositions of the invention may comprises fluoride sources such as sodium fluoride, stannous fluoride, sodium monofluorophosphate, zinc ammonium fluoride, tin ammonium fluoride, calcium fluoride, cobalt ammonium fluoride and mixtures thereof.

The invention includes agents for the remineralisation of teeth. The term "remineralisation" in the context of the present invention means the *in situ* generation of hydroxyapatite on teeth.

The agent for the remineralisation of teeth is a mixture of a calcium silicate and a phosphate source which, when delivered to the teeth results in the *in situ* generation of hydroxyapatite on teeth.

The amount of calcium silicatein the composition of the invention typically ranges from 1 to 30%, preferably from 5 to 20% based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

Mixtures of any of the above described materials may also be used.

The composition according the invention will comprise further ingredients which are common in the art, such as:
antimicrobial agents, e.g. chlorhexidine, sanguinarine extract, metronidazole, quaternary ammonium compounds, such as cetylpyridinium chloride; cetylpyridium chloride clay complex bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
hyaluronic acid;
amino acids such as arginine;
colouring agents;
pH-adjusting agents;
sweetening agents;
pharmaceutically acceptable carriers, e.g. starch, sucrose, water or water/alcohol systems etc.; surfactants, such as anionic, nonionic, cationic and zwitterionic or amphoteric surfactants; Humectants such as glycerol, sorbitol, propyleneglycol, xylitol, lactitol etc.;
binders and thickeners such as sodium carboxymethyl-cellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Example of the invention will now be illustrated by the following examples:

### Examples

**Table 1**

| **Ingredient** | **Wt. %** | **Wt. %** |
|---|---|---|
| | **Example A** | **Example 1** |
| Glycerin | To 100 | To 100 |
| PEG400 | | 2.5 |
| Carrageenan Kappa | 0.075 | 0.075 |
| Carrageenan Iota | 0.075 | 0.075 |
| SLS | 2 | 2 |
| Trisodium Phosphate anhydrous | 3.8 | 3.8 |
| Monosodium Phosphate anhydrous | 3.2 | 3.2 |
| Calcium silicate | 15 | 15 |
| Sorbosil AC33 | 2 | 2 |
| Sodium Saccharin | 0.22 | 0.22 |
| Sodium Monofluorophosphate | 1.11 | 1.11 |
| Flavour | 0.7 | 0.7 |

| **Example** | **Change in Storage modulus after heat ramp 25 to 60 to 25 degC / Pa** | **Change in yield stress after heat ramp 25-60-25 degC / Pa** |
|---|---|---|
| A | 421,000 | 2600 |
| 1 | 4,242 | 44.6 |

The above Examples demonstrate that the Example according to the invention remains relatively un-changed on storage compared to the comparative Example.

## Claims

1. A non-aqueous oral care composition comprising:
i) greater than 50 wt% of an organic polyol having 3 or more hydroxyl groups in the molecule;
ii) a carrageenan;
iii) a polyethylene glycol and/or polypropylene glycol having a melting point below 40°C;
iv) a mixture of a calcium silicate and a phosphate source which, when delivered to the teeth results in the *in situ* generation of hydroxyapatite on teeth
wherein the term non-aqueous means water is present in an amount no greater than 5wt% of the total composition.

2. A non-aqueous oral care composition according to claim 1 in which iii) is a polyethylene glycol having a melting point below 25°C.

3. A non-aqueous oral care composition according to claim 1 or claim 2 in which iii) has a melting point above -20°C.

4. A non-aqueous oral care composition according to any preceding claim in which the carrageenan comprises a 1:2 to 2:1 weight ratio of iota to kappa carrageenan.

5. A non-aqueous oral care composition according to any preceding claim in which the organic polyol is glycerol.

6. A non-aqueous oral care composition according to any preceding claim in which the level of carrageenan is from 0.05 to 1 wt% of the total composition, more preferably 0.08 to 0.5 wt% most preferably 0.05 to 0.25 wt%.

7. A non-aqueous oral care composition according to any preceding claim in which the level of polyethylene glycol and/or polypropylene glycol iii) is from 0.1 to 10 wt% of the total composition, more preferably 0.5 to 7wt%, most preferably 1 to 5 wt%.

8. A non-aqueous oral care composition according to any preceding claim in which the weight ratio of polyethylene glycol and/or polypropylene glycol iii) to carrageenan is from 1: 200 to 1:4, preferably from 1:1 to 1:40.

9. A non-aqueous oral care composition according to any preceding claim in which the level of polyol is greater than 65wt% of the total composition.

10. A non-aqueous oral care composition according to any preceding claim that is a toothpaste.

11. A non-aqueous oral care composition according to any preceding claim which further comprises an abrasive.

12. A non-aqueous oral care composition according to any preceding claim composition further comprising an anionic surfactant preferably sodium lauryl sulphate.

## Patentansprüche

1. Nichtwässrige Mundpflegezusammensetzung, umfassend:
i) mehr als 50 Gew.-% eines organischen Polyols mit 3 oder mehr Hydroxylgruppen im Molekül;
ii) ein Carrageen;
iii) ein Polyethylenglykol und/oder Polypropylenglykol mit einem Schmelzpunkt unter 40°C;
iv) eine Mischung aus einem Calciumsilikat und einer Phosphatquelle, die bei der Abgabe an die Zähne zur in situ-Bildung von Hydroxylapatit auf den Zähnen führt,
wobei der Begriff "nichtwässrig" bedeutet, dass Wasser in einer Menge von nicht mehr als 5 Gew.-% der Gesamtzusammensetzung vorhanden ist.

2. Nichtwässrige Mundpflegezusammensetzung nach Anspruch 1, wobei iii) ein Polyethylenglykol mit einem Schmelzpunkt unter 25°C ist.

3. Nichtwässrige Mundpflegezusammensetzung nach Anspruch 1 oder Anspruch 2, bei der iii) einen Schmelzpunkt über -20°C aufweist.

4. Nichtwässrige Mundpflegezusammensetzung nach einem vorhergehenden Anspruch, wobei das Carrageen ein Gewichtsverhältnis von Iota- zu Kappa-Carrageen von 1:2 bis 2:1 aufweist.

5. Nichtwässrige Mundpflegezusammensetzung nach einem vorhergehenden Anspruch, wobei das organische Polyol Glycerin ist.

6. Nichtwässrige Mundpflegezusammensetzung nach einem vorhergehenden Anspruch, wobei der Gehalt an Carrageen 0,05 bis 1 Gew.-% der Gesamtzusammensetzung beträgt, bevorzugter 0,08 bis 0,5 Gew.-% und höchst bevorzugt 0,05 bis 0,25 Gew.-%.

7. Nichtwässrige Mundpflegezusammensetzung nach einem vorhergehenden Anspruch, wobei der Gehalt an Polyethylenglykol und/oder Polypropylenglykol iii) 0,1 bis 10 Gew.-% der Gesamtzusammensetzung beträgt, bevorzugter 0,5 bis 7 Gew.-% und höchst bevorzugt 1 bis 5 Gew.-%.

8. Nichtwässrige Mundpflegezusammensetzung nach einem vorhergehenden Anspruch, wobei das Gewichtsverhältnis von Polyethylenglykol und/oder Polypropylenglykol iii) zu Carrageen zwischen 1:200 und 1:4 liegt, vorzugsweise zwischen 1:1 und 1:40.

9. Nichtwässrige Mundpflegezusammensetzung nach einem vorhergehenden Anspruch, wobei der Gehalt an Polyol mehr als 65 Gew.-% der Gesamtzusammensetzung beträgt.

10. Nichtwässrige Mundpflegezusammensetzung nach einem vorhergehenden Anspruch, die eine Zahnpasta ist.

11. Nichtwässrige Mundpflegezusammensetzung nach einem vorhergehenden Anspruch, die ferner ein Schleifmittel umfasst.

12. Nichtwässrige Mundpflegezusammensetzung nach einem vorhergehenden Anspruch, die ferner ein anionisches Tensid umfasst, vorzugsweise Natriumlaurylsulfat.

## Revendications

1. Composition de soin buccodentaire non aqueuse comprenant :
i) plus de 50 % en poids d'un polyol organique ayant 3 groupes hydroxyle ou plus par molécule ;
ii) une carraghénane ;
iii) un polyéthylèneglycol et/ou polypropylèneglycol ayant un point de fusion inférieur à 40 °C ;
iv) un mélange d'un silicate de calcium et d'une source de phosphate qui, lorsqu'il est délivré aux dents, a pour résultat la génération *in situ* d'hydroxyapatite sur les dents,
dans laquelle l'expression "non aqueuse" signifie que l'eau est présente en une quantité non supérieure à 5 % en poids de la composition totale.

2. Composition de soin buccodentaire non aqueuse selon la revendication 1, dans laquelle iii) est un polyéthylèneglycol ayant un point de fusion inférieur à 25 °C.

3. Composition de soin buccodentaire non aqueuse selon la revendication 1 ou la revendication 2, dans laquelle iii) a un point de fusion supérieur à -20 °C.

4. Composition de soin buccodentaire non aqueuse selon l'une quelconque des revendications précédentes, dans laquelle la carraghénane comprend un rapport des carraghénanes iota à kappa de 1/2 à 2/1.

5. Composition de soin buccodentaire non aqueuse selon l'une quelconque des revendications précédentes, dans laquelle le polyol organique est le glycérol.

6. Composition de soin buccodentaire non aqueuse selon l'une quelconque des revendications précédentes, dans laquelle le taux de carraghénane est de 0,05 à 1 % en poids, mieux encore de 0,08 à 0,5 % en poids, tout spécialement de 0,05 à 0,25 % en poids de la composition totale.

7. Composition de soin buccodentaire non aqueuse selon l'une quelconque des revendications précédentes, dans laquelle le taux de polyéthylèneglycol et/ou de polypropylèneglycol iii) est de 0,1 à 10 % en poids, mieux encore de 0,5 à 7 % en poids, tout spécialement de 1 à 5 % en poids de la composition totale.

8. Composition de soin buccodentaire non aqueuse selon l'une quelconque des revendications précédentes, dans lequel le rapport en poids du polyéthylèneglycol et/ou du polypropylèneglycol iii) à la carraghénane est de 1/200 à 1/4, de préférence de 1/1 à 1/40.

9. Composition de soin buccodentaire non aqueuse selon l'une quelconque des revendications précédentes, dans laquelle le taux de polyol est supérieur à 65 % en poids de la composition totale.

10. Composition de soin buccodentaire non aqueuse selon l'une quelconque des revendications précédentes, qui est une pâte dentifrice.

11. Composition de soin buccodentaire non aqueuse selon l'une quelconque des revendications précédentes, qui comprend en outre un abrasif.

12. Composition de soin buccodentaire non aqueuse selon l'une quelconque des revendications précédentes, comprenant en outre un tensioactif anionique, de préférence le laurylsulfate de sodium.
